# EUROPEAN PATENT APPLICATION

(11) **EP 2 524 985 A1**
(43) Date of publication of application: **21.11.2012**
(21) Application number: 11732686.8
(22) Date of filing: 17.01.2011
(51) Int. Cl.: D04H 1/46, D01C 3/00, C14C 3/02

(54) **RESTORING LEATHER AND METHOD FOR MAKING THE SAME**

(30) Priority: 17.06.2010 CN 201010211811; 16.01.2010 CN 201010300376
(71) Applicant: Zhejiang Hongzhan New Materials Co., Ltd, Tongxiang, Zhejiang 314500 (CN)
(72) Inventor: ZHANG, Liwen, Guangzhou Guangdong 510170 (CN)
(74) Representative: Ström & Gulliksson AB
(86) International application number: PCT/CN2011/070329
(87) International publication number: WO 2011/085696

(57) **Abstract**

Disclosed are a reducing leather and a method for preparing the same. The leather is mainly made by three-dimensionally interweaving the collagen fibres obtained through fibre disintegration of a leather material, with an apparent density of 0.35g/cm³-1.4g/cm³. The method for preparing the leather comprises the steps of preparing collagen fibres by fibre disintegration of a leather material, laying, spunlacing, and wet-hot shrinking setting. The reducing leather approaches to a natural leather in the weaving structure and the apparent density, and each of the strength criteria, as well as the wear resistance. The advantages of the leather are that the leather piece is big and regular so that it can be arbitrarily tailored, and the weaving apparent density is consistent. The scraps in the leather industry are mainly used as the raw materials for the reducing leather. The preparation method facilitates to reutilizing the resources in the leather industry.

## Description

Technical Field

This invention relates to leathers, particularly to a reducing leather and a method for preparing the same.

Background Art

The leather industry has been born for many years, in which the skins spudded from animals are utilized, making the same fleshed, unhaired, and degreased, then passing through the tanning, filling, dyeing, and fatliquoring processes to make the same generating a series of chemical reactions, and finally becoming a finished leather which not only have some resistances to acid, alkali, and wet-hot, but also have good softness and fullness. The true skins of animals are mainly made by interweaving the collagen fibres and a minor amount of elastic fibres and reticular fibres, wherein the collagen fibres occupy about 95% of the total tare weight, and in fact, the leather processing is subjecting the collagen fibres to a tanning modification to make their chemical properties generating some changes.

The applications of animal skins in the industries are mainly to produce leathers. In the leather producing process, due to various defeats present in the raw material skins, such as scalding markers, fat wrinkles, bur scrapes and the like, as well as other causes, ultimately only 25% - 40% of the raw skins are processed into the finished products, and the remainders become scraps, however, these scraps not only result in a pollution to the environment, but also at the same time result in a regret to humans as such a natural precious protein material can not be scientifically and reasonably utilized. Over the years, although the people have also carried out unceasing researches, developments and utilizations on these scraps, and made a minor amount of such materials obtaining some uses, however, the processing technology and the product quality is low, the application scope is small, and the materials are not really fully utilized.

The public specification of the Chinese invention patent application CN1779002A discloses a collagen fibre reducing leather based cloth and a method for preparing the same, which provide a method for effectively obtaining the collagen fibres from the skin materials and weaving them into a based cloth by a nonwoven technology, the based cloth obtained by this method has possessed a weaving structure similar to a natural leather, however, it's weaving structure is loose, the apparent density is only 0.25-0.3g/cm³, and the tearing strength of the based cloth of 500g /m² is still only about 8N, so it is just a base material, and is not suitable for commercial application without a further treatment. Due to the limits of the prior nonwoven process technologies, not only the collage fibres, when other chemical fibres and natural fibres are processed by the prior nonwoven technology, particularly the spunlacing process, the appearance densities of the products thereof are only lower than 0.25g/cm³, while the appearance densities of the leathers made from true skins are commonly between 0.6g/cm³-1.0g/cm³, and the tearing strengths of the true skins of 500g/cm² are generally ≥30N.

Contents of the Invention

Aiming at the problems present in the abovementioned prior art, the primary purpose of the present invention is to provide a reducing leather which approaches to the true skin in the weaving appearance density and each of the strength criteria, as well as the wear resistance.

The purpose of the present invention is also to provide a method for preparing the abovementioned reducing leather.

The purposes of the present invention are carried out by the following technical solutions:

first, the present invention provides a reducing leather, the reducing leather is mainly made by three-dimensionally interweaving the collagen fibres obtained through fibre disintegration of a leather material, with an appearance density of up to 0.35g/cm³ -1.4g/cm³. With regard to the determination method for the appearance density, the method mentioned in The People's Republic of China Light Industry Standard QB/T 2715-2005 "Leather Physical and mechanical testsDetermination of apparent density" can be chosen to carry out the determination. The leather can also comprise the retanning agents and fillers which are useful in the leather industry.

As a preference, the reducing leather of the present invention is consisted of the components at a weight proportion as follows:

collagen fibres obtained through fibre disintegration of a leather material 70% -98%

retanning agents and fillers 2% -30%

other fibre and non-fibre impurities blended non-artifically neglectable.

The retanning agents used in the abovementioned leather industry comprise inorganic tanning agents, such as chromium, zirconium, titanium, ferrum tanning agents, etc.; and organic tanning agents, such as aldehydes, vegetables, resins tanning agents, etc.; the fillers used comprise: fatliquoring agents, such as various animal oils, vegetable oils, mineral oils, and synthetic oils thereof, organosilicons, organofluorines, salts, such as a sulphate salt, a thiosulfate salt, a carbonate salt, a barium salt and a calcium salt.

The present invention also provides a method for preparing the abovementioned reducing leather, the core steps thereof comprising:

(1) preparing collagen fibres through fibre disintegration of a leather material;

(2) laying the collagen fibres;

(3) spunlacing so as to obtain a spunlacing leather material;

(4) subjecting the spunlacing leather material to wet-hot shrinking setting so as to obtain the reducing leather.

On the basis of the requirements, the leather material can be subjected to the routine treatments in the leather industry such as, retanning, filling, dyeing, softening, finishing, impregnating, coating, coating, laminating, sueding, etc.

The collagen fibres are obtained by a method for fibre disintegration of a leather material. If the fibres of the leather material is loosely woven, they can be obtained through the conventional technologies by opening and carding the raw material. In general, it is preferable to carry out fibre disintegration by either the liquid opening machine disclosed in the issued specification of the Chinese invention patent CN 100545329C or the method disclosed in the public specification of the Chinese invention patent application CN1779002A.

As a preference, the wet-hot shrinking setting in the step (4) is a water-boiling shrinking setting, a vapour shrinking setting or hot rolling shrinking setting.

The temperature for the wet-hot shrinking setting is generally between 60°C-105°C, as a preference, said temperature for the wet-hot shrinking setting is determined on the basis of the shrinkage temperature of the spunlacing leather material in the step (3), provided that the shrinkage temperature is N°C, then the temperature for the wet-hot shrinking setting is set between N°C-N°+10C; said shrinkage temperature of the spunlacing leather material is measured according to the method provided in the People's Republic of China Light Industry Standard QB/T 2713-2005 "Determination of the Shrinkage Temperature of Leather", wherein water is used as the detection solution.

In the wet-hot shrinking setting process in the step (4), it is preferable to use a leather water absorption salt solution which generates a water absorption on the collagen fibres in the spunlacing leather material to process the spunlacing leather material. After the collage fibres are processed with the leather water absorption salt solution, the shrinkage temperature will be significantly reduced, after the salts is removed, the shrinkage temperature can be recovered, therefore, in the wet-hot shrinking setting step of this technology, it can not only save the heat energy, but also make the collagen fibres in the spunlacing leather material far from the irreversible denaturation temperature. The leather water absorption salt is at least one of a lithium salt, a magnesium salt, a calcium salt, an iodide, a barium salt, and a thiocyanate salt, with a concentration range generally between 10wt% - 60wt%.

The usage for the water absorption salt solution is preferable to, but not limited to the two kinds as follows:

1. The spunlacing leather material in the step (3) is carried out an ambient temperature impregnation or a heating impregnation in a solution containing a leather water absorption salt solution to make the same shrinking setting; said temperature of the solution containing a leather water absorption salt is determined on the basis of said shrinkage temperature of the spunlacing leather material in step (3), provided that the shrinkage temperature is N₁°C, then the temperature for the wet-hot shrinking setting is set between N₁°C-N₁-80°C; said shrinkage temperature of the spunlacing leather material is measured according to the method provided in the People's Republic of China Light Industry Standard QB/T 2713-2005 "Determination of the Shrinkage Temperature of Leather ", wherein said leather water absorption salt solution is used as the detection solution.

2. First the spunlacing leather material in the step (3) is impregnated in a solution containing a leather water absorption salt, then carried out a vapour heating or a heat rolling treatment to make the same shrinking setting; the temperature for said vapour heating and heat rolling treatment is determined on the basis of said shrinkage temperature of the spunlacing leather material in the step (3), provided that the shrinkage temperature is N₂°C, then the temperature for the wet-hot shrinking setting is set between N₂°C-N₂-80°C; said shrinkage temperature of the spunlacing leather material is measured according to the method provided in the People's Republic of China Light Industry Standard QB/T 2713-2005 "Determination of the Shrinkage Temperature of Leather ", wherein said leather water absorption salt solution is used as the detection solution.

Through the wet-hot shrinking setting treatment in the step (4) of the present invention, the shrinkage ratios of the length and width of the material are generally between 20%-50%.

The reducing leather obtained from the present invention can be used in all of the fields that the true skin can be applied, such as shoes, clothing, suitcases and bags, sofa, etc.

As compared with the prior leather-based cloth, the present invention has the following advantages:

(1) Obtained in the present invention, is a reducing leather mainly made from the collage fibres obtained through fibre disintegration of a leather material, as raw materials, the leather is a reducing leather which approaches to a true skin in the weaving structure and the appearance density and each of the strength criteria as well as the wear resistance. As compared with a true skin, the advantages of the reducing leather are that the leather piece is big and regular so that it can be arbitrarily tailored, and the weaving appearance density is consistent, without a difference between various positions, when the thickness is 1.0, the tearing strength is more than 26N.

(2) In the method provided in the present invention, particularly a shrinking setting step is set on the basis of the wet-hot shrinking property possessed by the collagen fibres. If the collage fibres are only subjected to the processes in the laying and spunlacing steps, the reducing leather is inferior to a true skin in the weaving appearance density and each of the strength criteria, as well as the wear resistance, and the true skin feeling is not strong, however, through the shrinking setting in the present invention to make the fabric generate shrinking and curling, and generate further weaving and winding with the neighboring fibres and the branches thereof so as to form a greater braid angle, a product having a fibre weaving appearance density identical with that of a true skin is obtained. In the particular embodiments, the shrinkage temperatures of the material are used respectively on the basis of the different raw materials, the temperature for the shrinking setting is set between the shrinkage temperature thereof and that higher than the temperature by 10°C, so that the purpose for shrinking setting can be achieved without generating an irreversible denaturation on the collage fibres.

(3) As compared with the prior regenerating leather, the reducing leather product has a higher grade, a wider application scope, so that it can further solve more scraps in the leather industry, form a recycling and reutilization of the resources, fully exert the value of the collagen protein, a natural precious resource, and facilitate to achieving the transformation from the conventional leather industry to energy-saving and environment-friendly industry.

Particular Embodiments

The present invention is further described in detail by incorporating the embodiments below, but the implementations of the present invention are not limited to therein.

The core steps for preparing the reducing leather in the present invention comprise preparing collagen fibres through fibre disintegration, laying, spunlacing and shrinking setting. Now the preferably particular embodiments for each step are described respectively.

Preparation of collagen fibres: the scraps of the raw skin materials were put into a liquid opening machine to carry out fibre disintegration, wherein the liquid opening machine can be home-made, ie., at least more than one beaters and carding fibre devices were equipped within the container, the solution used in the liquid opening machine was water, 0.5wt%-5wt% an emulsified oil, 2wt%-51wt% of an antifoaming agent were added with a proportion of water into the liquid on the basis of the different leather materials, in the water within the abovementioned liquid opening machine the leather materials were beaten reciprocally by said beaters, making the fibres gradually loosing, separating, and keeping a length scope of 10 - 40mm, the separated fibres were pull out by the carding devices inside the machine, and discharged out of the machine. The fibres so formed were carried out baking and drying by a device or natural drying, then carried out opening by the opening machine at hand, so as to obtain the collagen fibres.

Laying: the collagen fibres obtained as above were formed into a collagen fibre web with a required thickness by a carding, laying technology or an air laid technology or a wet laid technology or a complex laid with two of those abovementioned forms in the prior processes.

Spunlacing: the abovementioned collage fibres were carried out a spunlacing by a spunlacing machine to make the collagen fibres and the branches thereof interlacing and interweaving so as to form a web weaving structure which is extremely similar to that of a true skin, referred to as a spunlacing leather material hereinafter.

Wet-hot shrinking setting: the wet-hot shrinking setting was divided into two cases that having or having not subjected to a leather water absorption salt treatment. The main differences between the two cases are that after the collagen fibres were subjected to a leather water absorption salt treatment, the shrinkage temperature were significantly reduced, so that the shrinking setting can be carried out at a lower temperature, which is more favorable to protecting the collagen fibres. Particularly when a higher concentration of a water absorption salt solution were used, the temperature for the shrinking setting of the spunlacing leather material is the lowest, which even can be selected at ambient temperature, and the upper limits are generally controlled around the shrinkage temperature of the spunlacing leather material before not subjecting to a water absorption salt treatment. In the temperature range as described above, the purposes for shrinking setting can be achieved all the time, without generating an irreversible denaturation on the collagen fibres. In the practical production, the span of the available temperature range of the shrinking setting temperature can be up to 80°C or more, depending on the particular production conditions.

The reducing leather processed by a leather water absorption salt can be desalted by washing with water

The abovementioned steps are only the core steps of the present invention, the leather material can also be carried out the treatments in the prior processes, such as retanning, filling, dyeing, softening, impregnating, coating, finishing, laminating, sueding, etc. These process steps can be interlaced into the different phases of the leather producing procedure on the basis of the requirements, rather than a successive treatment process only.

Embodiment 1

(1) Preparation of collagen fibres: the chromium-tanned true skins or the scraps thereof were put into a liquid opening machine (a device disclosed in the issued specification of the Chinese invention patent CN 100545329C, infra) to carry out fibre disintegration. The solution used in the liquid opening machine was water, and 2wt% an emulsified oil and 3wt% of an antifoaming agent with a proportion of water were added into the liquid. The fibres formed through fibre disintegration were carried out baking and drying by a device or natural drying, then were fully opened through a FA104 type hexa-roller opening machine, so as to obtain the collagen fibres.

(2) Laying: the collagen fibres obtained as above were formed into a collagen fibre web of 200g/m2 through a K12 air-laid machine.

(3) Spunlacing: the above collagen fibres were subjected to one plate spunlacing and five rotary-drum spunlacings, with the spunlacing presses of 60bar, 150bar, 200bar, 100bar and 80bar, respectively, making the collagen fibres and the branches thereof interlacing and interweaving so as to form a web weaving structure which is extremely similar to that of true skin, referred to as spunlacing leather material.

(4) Wet-hot shrinking setting: first the samples were taken and were detected by the mode in QB/T 2713-2005, and the shrinkage temperature of the spunlacing leather material were detected as 95°C. A single curtain impregnator in the prior art were used to carry out padding, the spunlacing leather material was impregnated into the padding device to carry out a high-temperature padding, with a temperature of the padding solution of 95°C. 10% of polyurethane was added into the padding solution.

(5) Drying and setting: a rolling drum drying device was selected to carry out a continuous "drying" ironing, with a temperature set at 80°C.

The reducing leather processed as the above process steps had a gram weight of 500g/cm² ± 10%, an appearance density of 0.56g/cm3, and a tearing strength of ≥28N.

Embodiment 2

Description of the process

The raw materials were the vegetable tanned true skins or the scraps thereof, after fibre disintegration, wet laid (100g/cm2), and spunlacing, then a spunlacing leather material was obtained, the shrinkage temperature of the spunlacing leather material was 80°C, a pass-through steaming cabinet was selected to carry out vapour heating so as to make the spunlacing leather material shrinking and setting, with a vapour temperature of 85°C. The detection method for the shrinkage temperature is identical with that in embodiment 1.

The rest processes were identical with those in embodiment 1.

The reducing leather processed as the abovementioned process steps had a gram weight of 230g/m² ± 8%, an appearance density of 0.38g/cm³, and a tearing strength of ≥20N.

Embodiment 3

Description of the process

The raw materials were wet blue leathers or the scraps thereof, after fibre disintegration, carding laid (300g/m²), and spunlacing, then a spunlacing leather material was obtained, the shrinkage temperature of the spunlacing leather material was 95°C. The above mentioned spunlacing leather material were impregnated into a padder to carry out padding, the temperature of the padding solution was set at 35°C, and 5wt% of tannin extract, 3wt% of amino silicone oil were added into the padding solution. After the leather materials were padded, they were carried out shrinking setting through a steaming cabinet, and the vapour temperature inside the steaming cabinet was set at 96°C. The detection method for the shrinkage temperature was identical with that in embodiment 1.

The rest processes were identical with those in embodiment 1.

The reducing leather processed with this process had a unit gram weight of 630g/m² ± 10 %, an appearance density of 0.42g/cm³, and a tearing strength of ≥30N.

Embodiment 4

The description of the process

The chromium tanned true skins or the scrapes thereof were selected as the raw materials. After fibre disintegration, through wet laid and air laid, overlaying together from top to bottom to form a complex fibre web (500g/m²), and spunlacing, then a spunlacing leather material was obtained, the shrinkage temperature of the sunlacing leather material was 100°C. First the samples of the spunlacing leather materials were taken, and detected by the method in QB/T 2713-2005 using a detector for the leather resistance to the wet-hot shrinkage temperature, wherein the difference was that the detection solution was 20wt% of a lithium salt aqueous solution, it was determined that the shrinkage temperature of the spunlacing leather material in this solution was reduced to 70°C. Then the spunlacing leather material was impregnated by a single curtain impregnator, the solution inside the impregnator was 20wt% of a lithium salt aqueous solution, after impregnation, the spunlacing leather material was carried out wet streaming by a continuous steam setting machine, with a temperature set at 70°C; or carried out hot rolling by a hot roller, with a temperature set at 80°C, making the same shrinking setting, then carried out desalting by washing with water, retanning, filling, and baking and drying.

The rest processes were identical with those in embodiment 1.

The reducing leather processed with this process had a unit gram weight of 1000g/m² ± 10%, an appearance density of 0.68g/cm³, and a tearing strength of ≥38N.

Embodiment 5

Description of the process:

The raw materials were the vegetable tanned true skins or the scraps thereof, after fibre disintegration, through wet laid and air laid, overlaying together from top to bottom to form a complex fibre web (500g/m²), and spunlacing, then a spunlacing leather material was obtained, and the shrinkage temperature of the spunlacing leather material was 100°C. First the samples of the spunlaicng leather material were taken, and detected by the method in QB/T 2713-2005 using a detector for the leather resistance to the wet-hot shrinkage temperature, wherein the difference was that the detection solution was 20wt% of a lithium salt aqueous solution, it was determined that the shrinkage temperature of the spunlacing leather material in this solution was reduced to 70°C. Then the spunlacing leather material was impregnated by a single curtain impregnator, the solution inside the impregnator was 20wt% of a lithium salt aqueous solution, after the impregnation, the spunlacing leather material was carried out wet steaming by a continuous steam setting machine, with a temperature set at 70°C; or carried out hot rolling by a hot roller, with a temperature set at 80°C, making the same shrinking setting, then carried out desalting by washing with water, retanning, filling, and baking and drying.

The rest processes were identical with those in embodiment 1.

The reducing leather processed with this process had a unit gram weight of 1000g/m² ±10%, an appearance density of 1.2g/cm³, and a tearing strength of ≥38N.

Embodiment 6

Description of the process

The raw materials were wet blue leathers or the scarps thereof, after fibre disintegration, through wet laid and air laid, overlaying together from top to bottom to form a complex fibre web (500g/m²), and spunlacing, then a spunlacing leather material was obtained, and the shrinkage temperature of the spunlacing leather material was 100°C. First the samples of the spunlacing leather material were taken, and detected by the method in QB/T 2713-2005 using a detector for the leather resistance to the wet-hot shrinkage temperature, wherein the difference was that the detection solution was 20wt% of a lithium salt aqueous solution, and it was determined that the shrinkage temperature of the spunlacing leather material in this solution was reduced to 70°C. Then the spunlacing leather material was impregnated by a single curtain impregnator, the solution inside the impregnator was 20wt% of a lithium salt aqueous solution, after impregnation, the spunlacing leather material was carried out wet steaming by a continuous steam setting machine, with a temperature set at 70°C, or carried out hot rolling by a hot roller, with a temperature set at 80°C, making the same shrinking setting, then carried out desalting by washing with water, retanning , filling, and baking and drying.

The rest processes were identical with those in embodiment 1.

The reducing leather processed with this process had a unit gram weight of 1000g/m² ± 10%, an appearance density of 1.4g/cm³, and a tearing strength of ≥38N.

The abovementioned embodiments are the preferable embodiments of the present invention, but the implementations of the present invention are not limited by the abovementioned embodiments, any other alternations, modifications, replacements, combinations, and simplifications made without departing from the spirit and principle of the present invention, such as using dry-hot shrinkage, mechanical pressurization, or using acid, alkali, other salts, oxidative, reductive, and organic solvents treatments, or using ultrasonic wave, mechanical vibration auxiliary treatments, and the like methods, during the shrinkage process, are all equivalent replacement means, which are all embraced in the protection scope of the present invention.

## Claims

1. A reducing leather, **characterized in that** said leather is mainly made by three-dimensionally interweaving the collagen fibres obtained through fibre disintegration of a leather material, and with an appearance density of up to 0.35g/cm³ - 1.4g/cm³.

2. A reducing leather according to claim 1, **characterized in that** the composition of said leather also comprises a retanning agent and a filler which are useful in the leather industry.

3. A reducing leather according to claim 2, **characterized by** comprising the components with a weight proportion as follows:
collagen fibres obtained through fibre disintegration of a leather material: 70% - 98%,
retanning agent and/or filler : 2% - 30%.

4. A method for preparing a reducing leather according to claim 1, **characterized by** comprising the steps of :
(1) preparing the collagen fibres through fibre disintegration of a leather material;
(2) laying the collagen fibres;
(3) spunlacing so as to obtain a spunlacing leather material;
(4) subjecting the spunlacing leather material to a wet-hot shrinking setting so as to obtain the reducing leather.

5. A method for preparing a reducing leather according to claim 4, **characterized by** also comprising one or more of retanning, filling, dyeing, softening, finishing, laminating, and sueding treatments.

6. A preparation method according to claim 4, **characterized in that** the wet-hot shrinking setting in the step (4) is a water-boiling shrinking setting, a vapour shrinking setting or a hot-rolling shrinking setting..

7. A preparation method according to claim 6, **characterized in that** the temperature for said wet-hot shrinking setting is determined on the basis of the shrinkage temperature of the spunlacing leather material in the step (3), provided that the shrinkage temperature is N°C, then the temperature for the wet-hot shrinking setting is set between N°C - N+10°C.

8. A preparation method according to claim 4, **characterized in that** the wet-hot shrinking setting in the step (4) is that the spunlacing leather material in the step (3) is subjected to an ambient temperature impregnation or a heating impregnation treatment in a solution containing a leather water absorption salt to make the same shrinking setting;
the temperature of said solution containing a leather water absorption salt is determined on the basis of the shrinkage temperature of the spunlacing leather material in the step (3), provided that the shrinkage temperature is N1°C, then the temperature for the wet-hot shrinking setting is set between N1°C-N1-80°C.

9. A preparation method according to claim 4, **characterized in that** the wet-hot shrinking setting in the step (4) is that first the spunlacing leather material in the step (3) is impregnated in the solution containing a leather water absorption salt, then carried out a vapour heating or a hot rolling treatment to make the same shrinking setting;
the temperature for said vapour heating or hot rolling treatment is determined on the basis of the shrinkage temperature of the spunlacing leather material in the step (3), provided that the shrinkage temperature is N2°C, then the temperature for the wet-hot shrinking setting is set between N2°C - N2-80°C.

10. A preparation method according to claim 8 or 9, **characterized in that** said leather water absorption salt is at least one of a lithium salt, a magnesium salt, a calcium salt, an iodide, a barium salt, and a thiocyanate salt.
